## Europäisches Patentamt

## European Patent Office

## Office européen des brevets

(11) Publication number: **0 106 812**
**B1**

(12) **EUROPEAN PATENT SPECIFICATION**

(45) Date of publication of patent specification: **12.07.89**

(51) Int. Cl.⁴: **A 61 K 37/62, A 61 K 37/54**

(21) Application number: **83830192.7**

(22) Date of filing: **05.10.83**

(54) **Pharmaceutical composition containing a fibrinolytic agent and a diffusion factor, useful for the treatment of the myocardium infarction.**

(30) Priority: **08.10.82 IT 4924182**

(43) Date of publication of application:
**25.04.84 Bulletin 84/17**

(45) Publication of the grant of the patent:
**12.07.89 Bulletin 89/28**

(84) Designated Contracting States:
**AT BE CH DE FR GB LI NL SE**

(56) References cited:
**GB-A-1 179 787
US-A-3 708 575
US-A-4 271 150**

**HEAEMOSTASIS, CH, vol. 12, no. 1-2, 1982. Abstracts of the seventh international Congress on thrombosis, October 13-16, 1982, abstract 13 Karger A.G. Basel, CH. F. BORRELLI et al.: "Effects of urokinase (UK)-hyaluronidase association on experimental myocardinal infarction (MI)"**

(73) Proprietor: **ISTITUTO FARMACOLOGICO SERONO
Via Casilina 125
I-00176 Roma (IT)**

(72) Inventor: **Borrelli, Francesco
via Adolfo Omodeo 14
I-00179-Rome (IT)**
Inventor: **Antonetti, Francesco
via Val Trompia 65
I-00141-Rome (IT)**

(74) Representative: **Bauer, Robert, Dipl.-Ing. et al
Boeters, Bauer & Partner Patentanwälte
Thomas-Wimmer-Ring 14
D-8000 München 22 (DE)**

Courier Press, Leamington Spa, England.

EP 0 106 812 B1

# EP 0 106 812 B1

## Description

The invention concerns a pharmaceutical composition useful for the treatment of myocardial infarction as defined below.

The term "myocardial infarction" describes the irreversible damage of the cells as well as the necrosis occurring as a consequence of a total or important reduction of the coronary flow feeding some areas of the cardiac muscle; moreover, it may be a consequence of an insufficient increase of the coronary flow with reference to an increased requirement of oxygen, as it may happen under some conditions of stress.

In almost all cases of persons suffering from an acute and/or previous myocardial infarction one finds in a more or less evident size a restriction of the inside diameter of the coronary artery, being the result of a coronary arteriosclerosis or of some other causes.

An exhaustive treatment of the factors involved in the cases of myocardial infarction and of the therapeutic means being nowadays in use for its care, its prevention and the reduction of the consequent irreversible damaging of the cardiac muscle, may be found in a recent review by J. T. Willerson and L. M. Buja bearing the title "Causes and course of the acute myocardial infarction" published in American Journal of Medicine 69, pages 902 to 914 (1980). This review resumes all current knowledge about the treatment and prevention of the myocardial infarction, and represents an exhaustive description of the state of the art. The importance of the coronary thrombosis in the genesis of the acute myocardial infarction has been and still is subject to dicussion. However, supported by the above-mentioned theoretical base, many authors have experimented with success the treatment with urokinase by means of intravenous infusion in the care of the primary myocardial acute infarction. The usefulness of the thrombolytic infarction treatment in its acute stage still is a controversial matter (Lancet, 1975, pages 624 to 626).

It has recently been shown that streptokinase administered through coronary infusion to some patients suffering myocardial infarction has been successful by causing the re-channeling of the obstructed coronary arteries. However, several secondary effects, such as arrhythmia, have been observed after the intracoronary administration of streptokinase.

Hyaluronidase, one of the first agents being indicated to change and to improve the consequence of the coronary arteries' obstruction, clearly reduces the extent of the experimentally induced necrosis. Such activity by hyaluronidase has been considered to be the result of three main mechanisms: The improved supply of nutritional substances to the myocardium; the increased washout of harmful metabolic substances, and the increase collateral haematic flow to the area with a reduced flow of blood.

US—A—3 708 575 describes therefore a composition useful for the treatment of cardiac arrythmias, thrombi, arteriosclerosis, cerebral infarctions and cerebral thrombosis, comprising glucuronoglycosamino-glycan hyaluronate lyase, which is administered by intravenous, intraarterial or intrathecal injection.

It is also known from US—A—4 271 150 to use a fibrinoolytic agent such as streptokinase or urokinase, for the treatment of thrombosis and cardial infarction. This reference discloses a preparation for oral administration containing urokinase in admixture with an effective urokinase stabilizing amount of a glycoprotein to stabilize urokinase against deactivation and enhance urokinase absorption from gastrointestinal tract.

It is further known from GB—A—1 179 787 to use a synergistic composition consisting of hyaluronate lyase and at least one proteinaceous compound, such as trypsin or streptokinase, for the treatment of allergic conditions. The streptokinase is present in an amount of not more than 10% by weight, referred to the weight of hyaluronidase.

The invention as claimed is intended to supply a new pharmaceutical combination which is advantageous in the treatment of myocardial infarction showing an overadditive decrease of respective enzyme values.

The invention consists in a pharmaceutical composition containing therapeutically efficient quantities of the fibrinolytic agent urokinase and the diffusion factor hyaluronidase as a combination for simultaneous or consecutive administration.

The protective and therapeutic effect of the inventive composition has been evaluated in the experimental myocardial infarction being induced in the rat by means of isoproterenol.

In this model, the myocardial lesions are the consequence of aggregation of the platelets within the coronary arteria, all caused by the catecholamines.

The physiologic and pathologic variations in the rats during the acute steps of the myocardial necrosis and the reinstatement are similar to those found in patients, that is for instance, modifications in the serum values of enzymes, lipids, catecholamines, steroids, and electrocardiographic alterations.

The experiments were carried out on rats of the Sprague-Dawley strain, at a weight of 225 to 260 grams.

Myocardial infarction was induced by subcutaneous administration of 2 doses of 85 mg/kg of isoproterenol chlorhydrate (administered volume: 1 ml/kg) within a time interval of 24 hours.

30 minutes after the first injection of isoproterenol, the animals were treated at random according to the following schedule:

1st group—physiologic solution 0.9% NaCl;

2nd group—hyaluronidase, 2500 I.U./kg;

2

3rd group—urokinase, 20.000 I.U./kg;
4th group—urokinase, 40.000 I.U./kg;
5th group—hyaluronidase (2.500 I.U./kg)+urokinase (20.000 I.U./kg);
6th group—hyaluronidase (2.500 I.U./kg)+urokinase (40.000 I.U./kg).

In a seventh group the animals did not receive isoproterenol and were treated with a normal physiological solution 0,9% (0,9% NaCl, control group).

The treatments were repeated six hours after the administration of isoproterenol, and later, at a time interval of 24 hours (immediately after the second injection of isoproterenol).

All treatments were performed by slow intravenous perfusion (0,8 ml/hour) into one of the tail veins.

The drugs were contained in a physiological solution (0.9% NaCl). To the animals of the 5th and 6th group, a solution containing both hyaluronidase and urokinase was injected.

Six months after the second injection of isoproterenol, two samples of blood were taken from the abdominal aorta of each ether-anesthetized animal. A sample placed into a plastic tube containing EDTA-Na, was used to determine the plasmatic levels of the following enzymes: Cardiac isoenzyme of the lactic dehydrogenase (LDH), glutamic oxalacetic transaminase (GOT) and creatinphosphokinase (CPK). The second sample was placed into a plastic test tube, containing, however, EDTA-Na plus acetyl salicylic acid (250 µg/ml of blood), and was submitted to the plasmatic thromboxane $B_2$ (TXB$_2$) determination by means of the radioimmunologic test method.

Immediately after the sampling, all animals were killed, the hearts were quickly removed, rinsed in a physiological solution, and carefully examined in order to ascertain the extent of the necrosis areas.

The results of the different treatments on the plasmatic levels of CPK, GOT and LDH are summarized in Table 1.

The administration of isoproterenol causes an increase in the values of CPK, GOT and LDH respectively to 93.4; 92.5 and 128 International Units per liter (I.U./l) in comparison with the normal corresponding values.

The administration of hyaluronidase alone causes a statistically significant reduction of 23.7% and 25.6% of the plasmatic levels of GOT and LDH respectively, whose values were increased by the action of isoproterenol. Similarly, a small reduction of the above-mentioned enzymes may be observed after a treatment with urokinase alone in a dose of 20.000 I.U./kg, whereas a more considerable inhibition is performed by the dosage of 40.000 I.U. kg (−20.4% for the GOT and −19.3% for the LDH).

The simultaneous administration of hyaluronidase (2.500 I.U./kg) with urokinase at the dosage of 20.000 I.U./kg performs an inhibition activity of the plasmatic GOT and LDH. This reduction is statistically significant and comparable to that performed by hyaluronidase alone. When hyaluronidase (2.500 I.U./kg) is administered in association with the higher dose of urokinase (40.000 I.U./kg), one obtains a further and statistically significant reduction of the plasmatic enzymes to 41,5% for GOT and 39,7% for LDH, respectively. As to the plasmatic levels of CPK, a significant reduction is obtained in a similar way with all treatments (−33% approximately), except the combination of hyaluronidase in a dose of 40.000 I.U./kg and of urokinase (2.500 I.U./kg) which reduces the CPK values by 41,3% compared to the values of the control animals.

TABLE 1

| Group No. | Treatments | | | No. of rats | Plasmatic levels of the cardiac enzymes | | | | | |
| --- | --- | --- | --- | --- | --- | --- | --- | --- | --- | --- |
| | Isoproterenol (mg/kg s.c.) | Hyaluronidase (UI/kg, i.v.) | Urokinase (UI/kg, i.v.) | | CPK (U/1) | | GOT (U/1) | | LDH (U/1) | |
| | X2 | X3 | X3 | | $\bar{X}\pm ES$ inhib. % | | $\bar{X}\pm ES$ inhib. % | | $\bar{X}\pm ES$ inhib. % | |
| 7 | — | — | — | 20 | 35,93+3,72 | — | 32,07±1,71 | — | 17,79±0,90 | — |
| 1 | 85 | — | — | 26 | 93,46±9,05 | — | 92,57±5,87 | — | 128,04±11,06 | — |
| 2 | 85 | 2500 | — | 15 | 63,86±7,97 | −33,0 | 70,57±9,52 | −23,7 | 95,29±0,37 | −25,6 |
| 3 | 85 | — | 20000 | 14 | 63,00±7,41 | −33,0 | 75,83±16,06 | −18,0 | 108,15±14,67 | −15,5 |
| 4 | 85 | — | 40000 | 15 | 62,33±5,70 | −33,3 | 73,69±7,13 | −20,4 | 103,26±18,30 | −19,3 |
| 5 | 85 | 2500 | 20000 | 14 | 63,77±7,06 | −32,0 | 66,73±6,26 | −27,9 | 94,17±12,37 | −26,4 |
| 6 | 85 | 2500 | 40000 | 17 | 54,81±6,16 | −41,3 | 54,09±6,32 | −41,5 | 77,14±15,25 | −39,7 |

EP 0 106 812 B1

In Table 2 the data referring to the $TXB_2$ are shown.

The administration of isoproterenol caused an increase of the $TXB_2$ plasmatic levels in the control group in comparison to normal values.

The treatment with hyaluronidase, urokinase or any combination of both brings the $TXB_2$ values back to the normal levels. A reduction of the necrosis areas was observed in all animals having been submitted to the treatment. Particularly a reduction of the necrosis of about 50% was observed in the animals having been treated with the combination of hyaluronidase and urokinase.

Finally, the survey of mortality as recorded during the experiments indicates a protective action of the treatment with the combination of hyaluronidase (2.500 I.U./kg) and urokinase (40.000 I.U./kg). As a matter of fact, only this treatment causes a sharp reduction of the mortality, compared to the control group, where the mortality was 32,5%. The results of the various treatments on the mortality of rats are summarized in Table 3.

TABLE 2

| Group No. | Treatments | | | No. of rats | Plasmatic levels | |
|---|---|---|---|---|---|---|
| | Isoproterenol (mg/kg, s.c.) | Hyaluronidase (UI/kg, e.v.) | Urokinase (UI/kg, e.v.) | | OF TXB $(pg/ml)^2$ | |
| | X2 | X3 | X3 | | $\overline{X} \pm ES$ | % variation |
| 7 | — | — | — | 20 | 427,18±2,02 | — |
| 1 | 85 | — | — | 26 | 635,82±87,40 | +48,84 |
| 2 | 85 | 2500 | — | 15 | 477,19±48,57 | +11,71 |
| 3 | 85 | — | 20000 | 14 | 455,00±44,58 | +6,51 |
| 4 | 85 | — | 40000 | 15 | 456,82±57,05 | +6,94 |
| 5 | 85 | 2500 | 20000 | 14 | 458,92±32,78 | +7,43 |
| 6 | 85 | 2500 | 40000 | 17 | 440,83±22,07 | +3,20 |

TABLE 3

| Group No. | Treatments | | | Total number of animals | No. of live animals | No. of dead animals | Rate of mortality |
| | Isoproterenol (mg/kg, s.c.) | Hyaluronidase (UI/kg, e.v.) | Urokinase (UI/kg, e.v.) | | | | |
|---|---|---|---|---|---|---|---|
| | X2 | X3 | X3 | | | | |
| 7 | — | — | — | 20 | 20 | 0 | 0 |
| 1 | 85 | — | — | 40 | 27 | 13 | 32,5 |
| 2 | 85 | 2500 | — | 20 | 15 | 5 | 25,0 |
| 3 | 85 | — | 20000 | 20 | 14 | 6 | 30,0 |
| 4 | 85 | — | 40000 | 20 | 15 | 5 | 25,0 |
| 5 | 85 | 2500 | 20000 | 20 | 14 | 6 | 30,0 |
| 6 | 85 | 2500 | 40000 | 20 | 17 | 3 | 15,0 |

EP 0 106 812 B1

# EP 0 106 812 B1

The above indicated results show that the intravenous administration of hyaluronidase and urokinase together reduces or causes some improvements in the infarction experimentally induced by isoproterenol. This observation is based principally on the study of the plasmatic levels of CPK, GOT, LDH and $TXB_2$, upon the macroscopic evaluation of the extent of the infarction-damaged areas, and on the mortality rate. In particular one may see that sharply and surprisingly higher protection effects are obtained by the simultaneous administration of hyaluronidase (2.500 I.U./kg) and urokinase (40.000 I.U./kg), as is shown by the GOT and LDH levels, and the mortality rate.

The pharmaceutical production of the composition in accordance with the invention is not particularly difficult. Since the substances are easily lyophilisable, this is the preferred form for stability reasons. Some excipients may be suitably used in the composition as a part thereof, and this addition does not change the inventive combination, consisting of the simultaneous or consecutive administration of a composition containing the fibrinolytic agent urokinase and the diffusion factor hyaluronidase each agent being separately or together in the medicament.

Based on the above shown results, it is clear that the inventive compositions containing urokinase and hyaluronidase, and particularly those containing them in a ratio higher than 8:1, the quantities being expressed in International Units of biologic activity of the relevant substances, are of particular advantage.

Within the scope of the present invention there are not only the pharmaceutical compositions containing both substances in one container, but also some particular forms of presentation supplying them separately and every form of actuation including and practically carrying out the above-mentioned therapeutical and pharmaceutical concept.

## Claims for the Contracting States: BE, NL, FR, GB, CH, SE, DE and LI

1. Pharmaceutical composition for the treatment of myocardial infarction containing therapeutically efficient quantities of the fibrinolytic agent urokinase and of the diffusion factor hyaluronidase as a synergistic combination for simultaneous or consecutive administration.

2. Pharmaceutical composition according to claim 1, wherein urokinase and hyaluronidase are contained in a ratio higher or equal to 8:1, the quantities being expressed in International Units.

3. Pharmaceutical composition according to claim 1 or 2 in lyophilized form in one or more ampoules for intravenous use.

4. Use of the fibrinolytic agent urokinase and of the diffusion factor hyaluronidase to produce a pharmaceutical composition according to claims 1 to 3.

## Claims for the Contracting State: AT

1. Process for the preparation of a pharmaceutical composition for the treatment of myocardial infarction containing therapeutically efficient quantities of the fibrinolytic agent urokinase and of the diffusion factor hyaluronidase as a synergistic combination for simultaneous or consecutive administration, which process comprises bringing said fibrinolytic agent urokinase and said diffusion factor hyaluronidase into a suitable pharmaceutical form.

2. A process according to claim 1 wherein urokinase and hyaluronidase are contained in a ratio higher or equal to 8:1, the quantities being expressed in International Units.

3. A process according to claim 1 or 2 wherein the pharmaceutical composition is in a lyophilized form in one or more ampoules for intravenous use.

## Patentansprüche für die Vertragsstaaten: BE, CH, DE, FR, GB, LI, NL, SE

1. Pharmazeutische Zusammensetzung für die Behandlung des Myocardinfarkts, enthaltend therapeutisch wirksame Mengen des fibrinolytischen Mittels Urokinase und des Diffusionsfaktors Hyaluronidase als synergistische Kombination für die gleichzeitige oder aufeinanderfolgende Verabreichung.

2. Pharmazeutische Zusammensetzung nach Anspruch 1, in der die Urokinase und die Hyaluronidase in einem Verhältnis über oder gleich 8:1 enthalten sind, wobei die Mengen in Internationalen Einheiten angegeben sind.

3. Pharmazeutische Zusammensetzung nach Anspruch 1 oder 2 in lyophilisierter Form in einer oder mehreren Ampullen für die intravenöse Verwendung.

4. Verwendung des fibrinolytischen Mittels Urokinase und des Diffusionsfaktors Hyaluronidase zur Herstellung einer pharmazeutischen Zusammensetzung nach den Ansprüchen 1 bis 3.

## Patentansprüche für den Vertragsstaat: AT

1. Verfahren zur Herstellung einer pharmazeutischen Zusammensetzung für die Behandlung des Myocardinfarkts, enthaltend therapeutisch wirksame Mengen des fibrinolytischen Mittels Urokinase und des Diffusionsfaktors Hyaluronidase als synergistische Kombination für die gleichzeitige oder aufeinanderfolgende Verabreichung, bei welchem Verfahren das fibrinolytische Mittel Urokinase und der Diffusionsfaktor Hyaluronidase in eine geeignete pharmazeutische Form gebracht werden.

2. Verfahren nach Anspruch 1, in dem die Urokinase und die Hyaluronidase in einem Verhältnis über oder gleich 8:1 verwendet werden, wobei die Mengen in Internationalen Einheiten angegeben sind.

3. Verfahren nach Anspruch 1 oder 2, in dem die pharmazeutische Zusammensetzung in lyophilisierter Form in einer oder mehreren Ampullen für die intravenöse Verwendung vorliegt.

**Revendications pour les Etats Contractants: BE, CH, DE, FR, GB, LI, NL, SE**

1. Composition pharmaceutique pour le traitement de l'infarctus du myocarde comprenant des quantités therapeutiques efficaces de l'agent fibrinolytique urokinase et du facteur de diffusion hyaluronidase en composition synergistique pour l'administration simultanée ou consécutive.

2. Composition pharmaceutique selon la revendication 1 comprenant l'urokinase et l'hyaluronidase dans und proportion au-dessus ou égale de 8:1, les quantités étant indiquées en unités internationales.

3. Composition pharmaceutique selon la revendication 1 ou 2 en forme lyophilisée dans une ou plusieurs ampoules pour l'usage intraveneux.

4. Utilisation de l'agent fibrinolytique urokinase et du facteur de diffusion hyaluronidase pour la préparation d'une composition pharmaceutique selon les revendications 1 à 3.

**Revendications pour l'Etat Contractant: AT**

1. Procédé de préparation d'une composition pharmaceutique pour le traitement de l'infarctus du myocarde comprenant des quantités therapeutiques efficaces de l'agent fibrinolytique urokinase et du facteur de diffusion hyaluronidase en composition synergistique pour l'administration simultanée ou consécutive, procédé dans lequel on met l'agent fibrinolytique urokinase et le facteur de diffusion hyaluronidase dans une forme pharmaceutique appropriée.

2. Procédé selon la revendication 1, dans lequel l'urokinase et l'hyaluronidase sont contenues dans une proportion au-dessus ou égale de 8:1, les quantités étant indiquées en unités internationales.

3. Procédé selon les revendications 1 ou 2, dans lequel la composition pharmaceutique est en forme lyophilisée dans une ou plusieurs ampoules pour l'usage intraveneux.